# EUROPEAN PATENT APPLICATION

(11) **EP 4 740 830 A1**
(43) Date of publication of application: **13.05.2026**
(21) Application number: 23944372.4
(22) Date of filing: 05.07.2023
(51) Int. Cl.: A61B 1/045

(54) **DIAGNOSTIC IMAGING ASSISTANCE DEVICE, DIAGNOSTIC IMAGING ASSISTANCE SYSTEM, AND DIAGNOSTIC IMAGING ASSISTANCE METHOD**

(71) Applicant: Olympus Medical Systems Corp., Tokyo 192-8507 (JP)
(72) Inventor: SUZUKI, Hiroshi, Hachioji-shi, Tokyo 192-8507 (JP); MIYAZAWA, Shingo, Hachioji-shi, Tokyo 192-8507 (JP); MATSUSHITA, Akira, Hachioji-shi, Tokyo 192-8507 (JP)
(74) Representative: Gunzelmann, Rainer
(86) International application number: PCT/JP2023/025015
(87) International publication number: WO 2025/009124

(57) **Abstract**

An image diagnosis assistance apparatus 5 includes an image selector 511 configured to select, as a diagnosis image, any one of a plurality of images that contain a same subject and on which sets of processing different from one another are executed; and an estimator 512 configured to, by executing an estimation process on the diagnosis image using a trained model, estimate a diagnostic candidate area serving as a diagnostic candidate in the diagnosis image and output reliability of the diagnostic candidate area. The image selector 511 is configured to select any one of the plurality of images as the diagnosis image based on the reliability of the diagnostic candidate area.

## Description

### Field

The present invention relates to an image diagnosis assistance apparatus, and an image diagnosis assistance method.

### Background

In recent years, in the field of medicine, image recognition techniques based on AI (Artificial Intelligence) have been proposed (for example, refer to Patent Literature 1).

The technique described in Patent Literature 1 executes an estimation process using a trained model on a captured image that is captured with an endoscope, thereby estimating a diagnostic candidate area, such as a lesion, or the like, in the captured image.

### Citation List

### Patent Literature

Patent Literature 1: Japanese Patent No. 6952214 Summary

### Technical Problem

With image recognition techniques based on AI, however, sufficient performance is not delivered depending on the definition of a captured image that is input and it is not possible to accurately estimate a diagnostic candidate area, such as a lesion, in some cases. In other words, it is not possible to provide an image suitable for diagnosis in some cases.

The present invention was made in view of the above-described circumstances and an object of the present invention is to provide an image diagnosis assistance apparatus, an image diagnosis assistance system, and an image diagnosis assistance method that make it possible to provide an image suitable for diagnosis.

### Solution to Problem

To solve the problem described above and to achieve the object, an image diagnosis assistance apparatus according to the present invention includes: an image selector configured to select, as a diagnosis image, any one of a plurality of images that contain a same subject and on which sets of processing different from one another are executed; and an estimator configured to, by executing an estimation process on the diagnosis image using a trained model, estimate a diagnostic candidate area serving as a diagnostic candidate in the diagnosis image and output reliability of the diagnostic candidate area. The image selector is configured to select any one of the plurality of images as the diagnosis image based on the reliability of the diagnostic candidate area.

An image diagnosis assistance system according to the present invention includes: an imaging device configured to generate a captured image by capturing an image of a subject; and an image diagnosis assistance apparatus configured to process the captured image. The image diagnosis assistance apparatus includes: an image selector configured to select, as a diagnosis image, any one of a plurality of images that contain a same subject and that are obtained by executing sets of processing different from one another are executed on the captured image; and an estimator configured to, by executing an estimation process on the diagnosis image using a trained model, estimate a diagnostic candidate area serving as a diagnostic candidate in the diagnosis image and output reliability of the diagnostic candidate area. The image selector is configured to select any one of the plurality of images as the diagnosis image based on the reliability of the diagnostic candidate area.

An image diagnosis assistance method according to the present invention is an image diagnosis assistance method that an image diagnosis assistance apparatus executes. The method includes: a step of selecting, as a diagnosis image, any one of a plurality of images that contain a same subject and on which sets of processing different from one another are executed; and a step of, by executing an estimation process on the diagnosis image using a trained model, estimating a diagnostic candidate area serving as a diagnostic candidate in the diagnosis image and outputting reliability of the diagnostic candidate area. The step of selecting the diagnosis image includes selecting any one of the plurality of images as the diagnosis image based on the reliability of the diagnostic candidate area.

### Advantageous Effects of Invention

According to the image diagnosis apparatus, the image diagnosis assistance system, and the image diagnosis assistance method, it is possible to provide an image suitable for diagnosis.

### Brief Description of Drawings

FIG. 1 is a diagram illustrating a configuration of an endoscope system according to an embodiment.
FIG. 2 is a diagram illustrating the configuration of the endoscope system according to the embodiment.
FIG. 3 is a diagram schematically illustrating a function of a controller.
FIG. 4 is a flowchart illustrating an image diagnosis assistance method.
FIG. 5 is a diagram illustrating a specific example of a display image.
FIG. 6 is a diagram illustrating a specific example of the display image.
FIG. 7 is a diagram illustrating a specific example of the display image.
FIG. 8 is a diagram illustrating a specific example of the display image.
FIG. 9 is a diagram illustrating Modification 1 of the embodiment.
FIG. 10 is a diagram illustrating Modification 2 of the embodiment.
FIG. 11 is a diagram illustrating Modification 3 of the embodiment.

### Description of Embodiments

A mode for carrying out the present invention (embodiment below) will be described below with reference to the drawings. Note that the embodiment described below do not limit the present invention. Furthermore, in the illustration of the drawings, the same reference numerals are assigned to the same parts.

### Configuration of Endoscope System

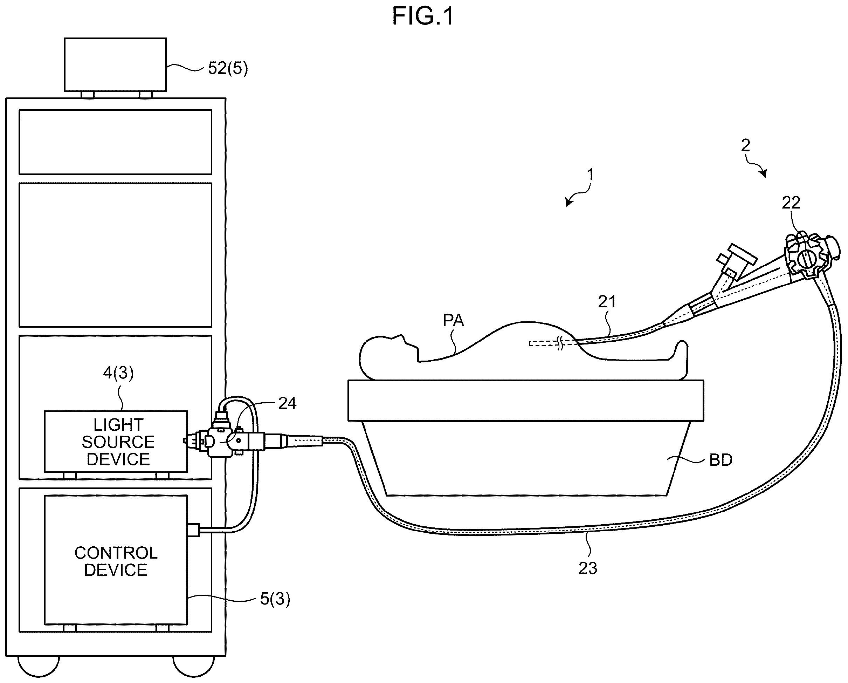

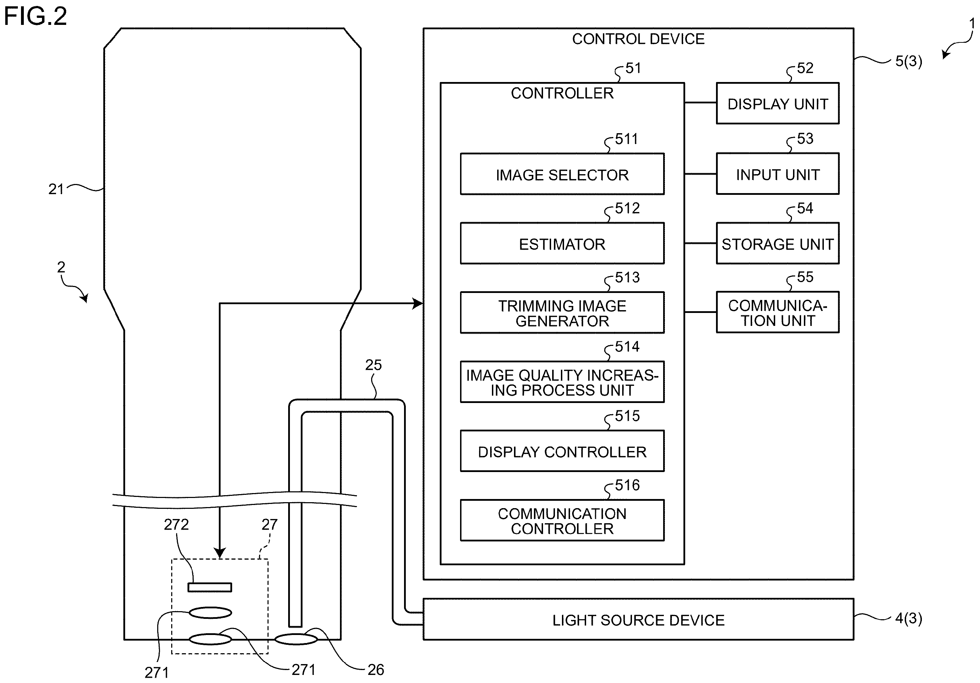

FIG. 1 and FIG. 2 are diagrams illustrating a configuration of an endoscope system 1 according to the embodiment.

The endoscope system 1 corresponds to an image diagnosis assistance system according to the present invention. The endoscope system 1 is a system that is used in the field of medicine and that observes a patient PA (FIG. 1) who is a patient on a bed BD (FIG. 1) in vivo (the large intestine in the embodiment). The endoscope system 1 includes an endoscope 2 and a processing device 3 as illustrated in FIG. 1 and FIG. 2.

The endoscope 2 corresponds to an imaging apparatus according to the present invention. In the present embodiment, the endoscope 2 is what is referred to as a flexible endoscope. The endoscope 2 is partly inserted into the body of the patient PA, captures an in-vivo image, and outputs an image signal that is generated by the image capturing. As illustrated in FIG. 1 and FIG. 2, the endoscope 2 includes an insertion portion 21, an operation unit 22, a universal cord 23, and a connector 24. Note that, in FIG. 2, for convenience of description, illustration of the operation unit 22, the universal cord 23, and the connector 24 is omitted.

The insertion portion 21 is a part that is flexible at least partly and that is inserted into the body of the patient P. In the insertion portion 21, as illustrated in FIG. 2, a light guide 25, an illumination lens 26, and an imaging unit 27 are arranged.

The light guide 25 is laid from the insertion portion 21 to the connector 24 via the operation unit 22 and the universal cord 23. One end of the light guide 25 is positioned in a distal end portion in the insertion portion 21. In the state where the endoscope 2 is connected to the processing device 3, the other end of the light guide 25 is positioned in the processing device 3. The light guide 25 transmits light that is supplied from a light source device 4 in the processing device 3 from the other end to the one end.

The illumination lens 26 is opposed to the one end of the light guide 25 in the insertion portion 21. The illumination lens 26 applies light that is transmitted by the light guide 25 to the internal body of the subject PA.

The imaging unit 27 is arranged in the distal end portion in the insertion portion 21. The imaging unit 27 captures an in-vivo image of the subject PA and outputs an image signal that is generated by the image capturing. As illustrated in FIG. 2, the imaging unit 27 includes a lens unit 271 and an imaging device 272.

The lens unit 271 takes in feedback light (a subject image) of the light that is applied from the illumination lens 26 to the internal body of the subject PA and forms the subject image on a light receiving surface of the imaging device 272.

The imaging device 272 is configured using a charge coupled device (CCD), a complementary metal oxide semiconductor (CMOS), or the like, that receives light of a subject image and converts the light into an electric signal and the imaging device 272 captures the subject image and thereby generates an image signal. Note that an image signal that is generated by the imaging unit 27 is referred to as a captured image.

The operation unit 22 is connected to a proximal end portion of the insertion portion 21. The operation unit 22 receives various types of operations on the endoscope 2.

The universal cord 23 is a cord that extends from the operation unit 22 in a direction different from the direction in which the insertion portion 21 extends and in which a signal lie that electrically connects the imaging unit 27 and a control device 5 in the processing device 3 and a light guide are arranged.

The connector 24 is set at an end of the universal cord 23 and is detachably connected to the processing device 3.

The processing device 3 includes the light source device 4 and the control device 5 as illustrated in FIG. 2.

Under the control of the control device 5, the light source device 4 supplies light to the other end of the light guide 25. In the embodiment, the light source device 4 emits white light as light of a first wavelength band. Note that the light source device 4 may be configured to be able to emit, as light of a second wavelength band different from the first wavelength band, excitation light that excites a fluorescent agent, such as indocyanine green, a narrow-band light used in NBI (Narrow Band Imaging), or the like.

The control device 5 corresponds to the image diagnosis assistance apparatus according to the invention. The control device 5 includes a controller 51, a display unit 52, an input unit 53, a storage unit 54, and a communication unit 55 as illustrated in FIG. 2.

The controller 51 is configured by including a controller, such as a CPU (Central Processing Unit) or a MPU (Micro Processing Unit), or an integrated circuit, such as an ASIC (Application Specific Integrated Circuit) or a FPGA (Field Programmable Gate Array), and controls entire operations of the endoscope system 1. The controller 51 has functions serving as an image selector 511, an estimator 512, a trimming image generator 513, an image quality increasing process unit 514, a display controller 515, and a communication controller 516 as illustrated in FIG. 2.

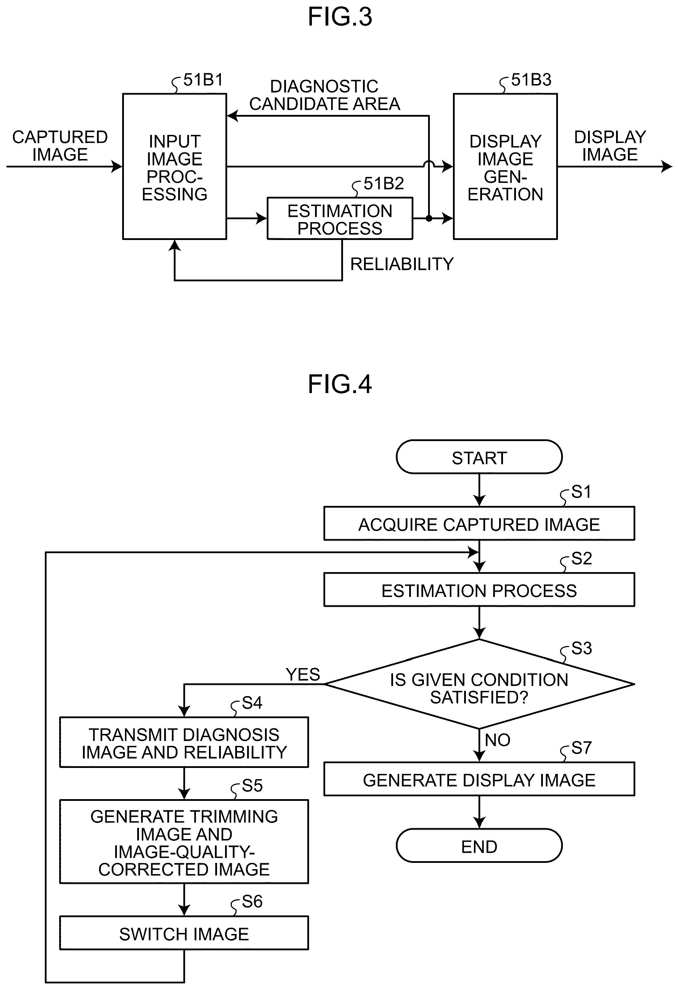

FIG. 3 is a diagram schematically illustrating a function of the controller 51.

In FIG. 3, "input image processing" denoted by a reference numeral 51B1 and in which the captured image (an endoscope image) is input includes the image selector 511, the trimming image generator 513, and the image quality increasing process unit 514. In FIG. 3, an "estimation process" denoted by a reference numeral 51B2 includes the estimator 512. Furthermore, in FIG. 3, "display image generation" denoted by a reference numeral 51B3 includes the display controller 515.

Note that the functions of the image selector 511, the estimator 512, the trimming image generator 513, the image quality increasing process unit 514, the display controller 515, and the communication controller 516 will be described in "Image Diagnosis Assistance Method" below.

The display unit 52 corresponds to a notification unit according to the invention. The display unit 52 is a LCD (Liquid Crystal Display) a EL (Electro Luminescence) display, or the like, and displays a display image that is generated by the controller 51 under the control of the controller 51.

The input unit 53 corresponds to an operation receiver according to the invention. The input unit 53 is configured using a keyboard, a mouse, a switch, a touch panel, or the like, and receives a user operation performed by a user, such as a practitioner. The input unit 53 outputs an operation signal corresponding to the user operation to the controller 51.

The storage unit 54 stores various types of programs that the controller 51 executes, information that is necessary for a process performed by the controller 51, etc.

The communication unit 55 is connected to an external device such that communication is enabled. The communication unit 55 transmits given information (data) to the external device under the control of the controller 51.

### Image Diagnosis Assistance Method

An image diagnosis assistance method that the control device 5 described above executes will be described next with reference to FIG. 3 and FIG. 4.

FIG. 4 is a flowchart illustrating the image diagnosis assistance method.

First of all, the image selector 511 acquires a captured image that is generated by the imaging unit 27 by, in a state where white light that is light of the first wavelength band from the light source device 4 is applied to the internal body of the subject PA, capturing an image of feedback light (subject image) of the white light from the internal body (step S1). The captured image corresponds to a first captured image according to the invention. The image selector 511 selects the captured image as a diagnosis image and inputs the diagnosis mage to the estimator 512.

After step S1, the estimator 512 executes the estimation process on the diagnosis image using an estimation process trained model and thereby estimates a diagnostic candidate area serving as a diagnostic candidate in each given area in the diagnosis image and outputs reliability of the diagnostic candidate area (step S2).

The reliability of the diagnostic candidate area is a value representing a level of reliability.

Specifically, reliability is a value representing accuracy of recognition of the image in the diagnostic candidate area and is also referred to as an index indicating a value representing a predicted possibility that an object to be imaged belongs to a specific class. It is possible to determine whether the object is recognized accurately in the area from the reliability of the diagnostic candidate area.

The estimation process trained model corresponds to a trained model according to the invention. The estimation process trained model is stored in the storage unit 54 previously. Specifically, a training process is executed repeatedly on a training model using a plurality of sets of training images and training data where a set includes a training image and a set of training data and the estimation process trained model is the training model after the training. The training image is a captured image obtained by capturing an internal image of a living body. The training data is data with annotations of a classification class, a right position, and a size of a lesion, or the like, in the training image. The training model used for the training process is, for example, a CNN (Convolutional Neural Network). The estimation process trained model contains a weight file (training parameters) including a weight value and a bias value of each layer of the CNN.

The neural network used for the training process to generate the estimation process trained model is not limited to the CNN, and other neural networks may be employed. For example, neural networks, such as a DNN (Deep Neural Network), Transformer, and GAN (Generative Adversarial Network), may be employed as appropriate. It is possible to employ known various learning algorithms as an algorithm of machine leaning in the neural network. For example, it is possible to employ a supervised learning algorithm using an error back-propagation algorithm.

After step S2, based on the reliability of the diagnostic candidate area that is output from the estimator 512 at step S2, the image selector 511 determines whether a given condition is met for a given number of times or for a given time (step S3).

It is possible to exemplify the following condition as the given condition used at step S3.

The given condition is that there is an "area with a possibility of being an abnormal portion" in an area in the diagnosis image.

The "area with a possibility of being an abnormal portion" indicates an area of which reliability is under a first threshold and is at or above a threshold lower than the first threshold. An "area that is a normal portion" indicates an area of which reliability is under the second threshold. Furthermore, the "area that is an abnormal portion" described below indicates an area of which reliability is at or above the first threshold.

The normal estimation process estimates whether it is a normal portion or an abnormal portion according to one threshold. Specifically, the reliability of the area is at or above the first threshold, it determines that it is "an area that is an abnormal portion" and, when the reliability of the area is under the first threshold, it determines that it is "an area that is a normal portion". In other words, an area under the first threshold is determined as being "an area that is a normal portion" in the same manner regardless of the magnitude of the value. On the other hand, in the estimation process, an area of which reliability is under the first threshold but is close to the first threshold, that is, "and area that is determined as being a normal portion but is with a possibility of being an abnormal portion" is sometimes overlooked. For example, there is a possibility that an accurate estimation process cannot be performed because the definition of the image that is to the estimation process is low. In this case, the area is overlooked although the area is an abnormal portion originally. The invention sets the second threshold that is a value smaller than the first threshold to avoid that risk. A configuration of, when it is under the first threshold but is at or above the second threshold, determining that it is "an area with a possibility that it is an abnormal portion", changing a feature of the image that is input to the estimation process, and performing the estimation process, is employed.

When it is determined that the given condition is met (Step S3: Yes), the communication controller 516 transmits the diagnosis image containing "an area with a possibility of being an abnormal portion" and the reliability of each given area in the diagnosis image to the external device from the communication unit 55 (step S4).

After step S4, the controller 51 (the trimming image generator 513 and the image quality increasing process unit 514) generates a trimming image and an image-quality-corrected image (step S5). The captured image that is acquired at step S1 and the trimming image and the image-quality-corrected image that are generated at step S5 correspond to "a plurality of images that contain the same subject and on which processes different from each other are executed" according to the invention.

Specifically, at step S5, the trimming image generator 513 generates a trimming image in which an area containing "an area with a possibility of being an abnormal portion" in the diagnosis image is enlarged. By executing an image quality increasing process on the trimming image using an image quality increasing process trained model, the image quality increasing process unit 514 generates an image-quality-corrected image with image quality that is increased as if the image is generated by an endoscope that generates a high-quality captured image (referred to as a high-definition endoscope below). Note that the image quality increasing process may be performed by, for example, a super resolution process or a classic image processing (such as tone processing, edge enhancement processing, frequency filter processing) other than the above-described AI processing.

The image quality increasing process trained model is stored in the storage unit 54 previously. Specifically, a training process is executed repeatedly on a training model using a plurality of sets of training images and training data where a set includes a training image and a set of training data and the image quality increasing process trained model is the training model after the training. The training image is an image obtained by lowering the quality of the captured image that is generated by the high-definition endoscope (referred to as a high image quality image below) according to the trimming image. The training image is a high image quality image. The training model that is used for the training process is, for example, a CNN. The image quality increasing process trained model contains a weight file (training parameters) including a weight value and a bias value of each layer of the CNN.

The neural network used for the image quality increasing process trained model is not limited to the CNN, and other neural networks may be employed. It is possible to employ known various learning algorithms as an algorithm of machine leaning in the neural network. For example, it is possible to employ a supervised learning algorithm using an error back-propagation algorithm.

After step S5, the image selector 511 switches the diagnosis image to one of the trimming image and the image-quality-corrected image that are generated at step S5 (step S6). For example, the image selector 511 selects an image that is set previously in a user operation on the input unit 53 as a diagnosis image from the trimming image and the image-quality-corrected image that are generated at step S5. The image selector 511 causes the switched diagnosis image (the trimming image or the image quality corrected image) to be input to the estimator 512. In other words, the controller 51 returns to step S2. For convenience of description, FIG. 4 and the description above have been given; however, while the image has to keep moving as a moving image in display image generation, the image that is input to the estimation process may be an image different from the display image. In other words, the image processing for the estimation process only has to move in the background and processing in branches as in FIG. 4 is not necessary practically.

When it is determined that the given condition is not satisfied (step S3: No), the display controller 515 generates a display image for the display unit 52 to display (step S7).

Details of the display image will be described in "Specific Example of Display image" described below.

### Specific Example of Display Image

A specific example of the display image that is displayed on the display unit 52 will be described next.

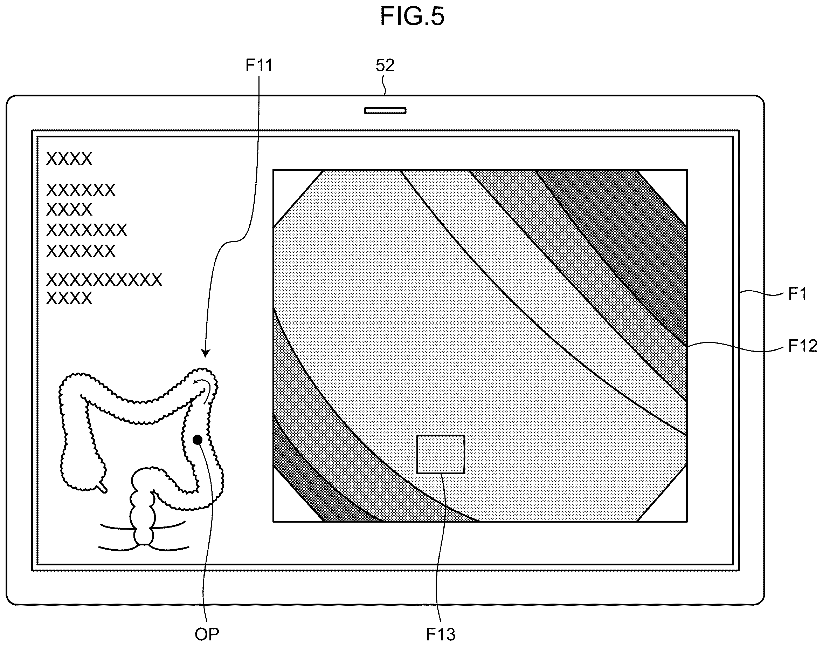

FIGS. 5 to 8 are diagrams illustrating the specific example of the display image.

For example, the display controller 515 generates a display image F1 illustrated in FIG. 5 in the above-described image diagnosis assistance method. The display controller 515 causes the display unit 52 to display the display image F1.

As illustrated in FIG. 5, the display image F1 contains an observation position image F11 and a diagnosis image F12.

The observation position image F11 is an image obtained by superimposing a current observation position (the distal end position of the insertion portion 21) OP onto an image representing a shape of a subject to be observed (the large intestine in the embodiment).

A diagnosis image F12 is the image (the captured image, the trimming image, or the image quality corrected image) that is selected as a diagnosis image by the image selector 511. In other words, when the diagnosis image is switched by the image selector 511 (step S6), the display controller 515 switches the diagnosis image F12 on the display image F1 to the switched diagnosed image. When it is determined by the estimation process that there is "an area that is an abnormal portion" in the diagnosis image F12, the display controller 515 superimposes the identification information F13 (FIG. 5) that identifies an area corresponding to the "area that is an abnormal portion" on the diagnosis image F12. In other words, the display controller 515 corresponds to a notification controller according to the invention.

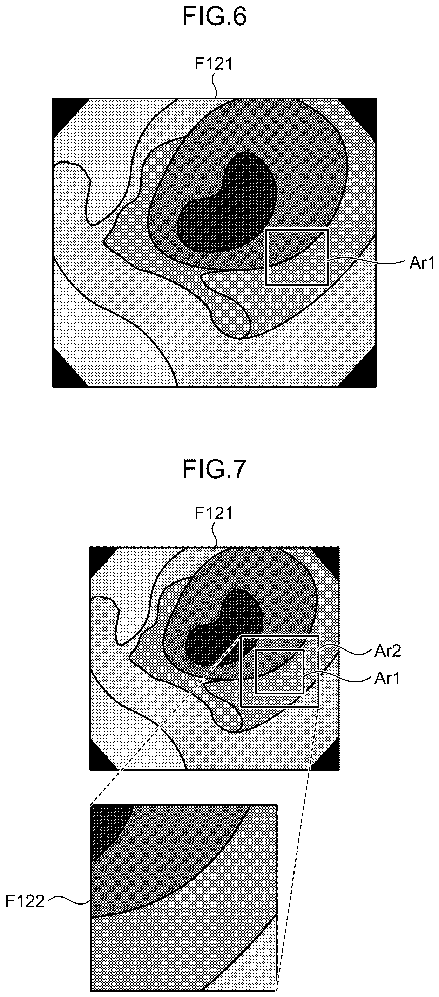

For example, the diagnosis image F121 illustrated in FIG. 6 is a captured image that is determined as satisfying the given condition at step S3. In the diagnosis image F121, an area Ar1 is "an area with a possibility of being an abnormal portion". In the case of the diagnosis image F121, because it is determined that the given condition is satisfied at step S3, a trimming image (or an image-quality-corrected image) F122 (FIG. 7) in which an area Ar2 containing an area Ar1 is enlarged is generated (step S5). The trimming image (or the image-quality-corrected image) F122 is then selected as a diagnosis image (step S6) and is input to the estimator 512. The diagnosis image F12 of the display image F1 is switched to the trimming image (or the image-quality-corrected image) F122.

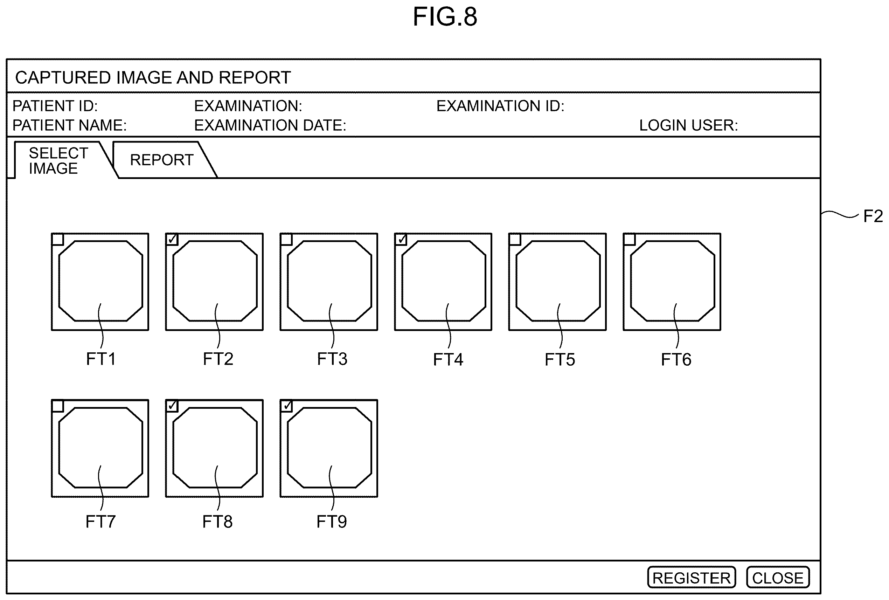

For example, the display controller 515 generates a display image F2 illustrated in FIG. 8. The display controller 515 causes the display unit 52 to display the display image F2.

When it is determined by the estimation process that there is "an area that is an abnormal portion" in the diagnosis image F12, the controller 51 stores the diagnosis image F12 sequentially in the storage unit 54.

The display controller 515 then generates a display image F2 displaying thumbnail images FT1 to FT9 of a plurality of diagnosis images F12 stored in the storage unit 54 in a list.

According to the embodiment described above, the following effects are achieved.

In the control device 5 according to the embodiment, the image selector 511 selects any one of the captured image that is acquired at step S1 and the trimming image and the image-quality-corrected image that are generated at step S5 as a diagnosis image based on reliability of a diagnostic candidate area. The image selector 511 causes the diagnosis image to be input to the estimator 512.

Accordingly, according to the control device 5 according to the embodiment, it is possible to cause a high-definition image to be input to the estimator 512, accurately estimate a diagnostic candidate area, such as a lesion, and provide an image suitable for diagnosis.

In order to increase accuracy of the estimation process in general, it is necessary to prepare suitable training data and repeatedly perform training. On the other hand, the present configuration appropriately and adaptively switches the image that is input to the estimation process according to the reliability that is output in the estimation process, thereby making it possible to increase accuracy of the estimation process without re-training even in a situation where the input image changes to an image unsuitable for the estimation process.

In the control device 5 according to the present embodiment, the display controller 515 superimposes identification information F13 that identifies an area corresponding to "an area that is an abnormal portion" from other areas on the diagnosis image F12.

This enables a user, such as a practitioner, to make a diagnosis appropriately based on an image obtained by superimposing the identification information F13 on the diagnosis image F12.

In the control device 5 according to the embodiment, the communication controller 516 causes the diagnosis image containing "an area with a possibility of being an abnormal portion" and the reliability of each given area in the diagnosis image from the communication unit 55 to the external device (step S4).

Thus, in the external device, by performing the training process again using the diagnosis image, it is possible to generate an estimation process trained model that makes it possible to accurately estimate a lesion, or the like again.

In the control device 5 according to the embodiment, the image selector 511 determines whether the given condition is satisfied for a given number of times or for a given time based on the reliability of the diagnostic candidate area that is output from the estimator 512 at step S2.

For this reason, the diagnosis image is not instantaneously switched when it is determined by error that the given condition is satisfied only once and it is possible to inhibit false detection.

### Other Embodiments

Modes for carrying out the invention have been described and the present invention should not be limited by only the above-described embodiment.

In the above-described embodiment, the image diagnosis assistance apparatus according to the invention is installed in the endoscope system 1 in which the insertion portion 21 is configured using a flexible endoscope; however, the system is not limited to this. For example, the image diagnosis assistance apparatus according to the invention may be installed in an endoscope system in which the insertion portion 21 is configured using a rigid endoscope. The image diagnosis assistance apparatus according to the invention may be installed in a medical observation system, such as a surgical microscope that enlarges and observes a given viewing filed area in the body of a subject (living body) or a subject surface (living-body surface) (for example, refer to Japanese Laid-open Patent Publication No. 2016-42981).

In the above-described embodiment, at least two of the captured image that is acquired at step S1, the trimming image and the image-quality-corrected image that are generated at step S5, and a second captured image to be described below may be contained as "a plurality of images that contain the same subject and on which processes different from each other are executed" according to the invention.

The second captured image is a captured image that is generated by the imaging unit 27 by, in a state where light of a second wavelength band (excitation light or narrow-band light) from the light source device 4 is applied to the internal body of the subject PA, capturing an image of feedback light (such as fluorescence) of the light of the second wavelength band from the internal body.

In the above-described embodiment, the display unit 52 is employed as the notification unit according to the invention; however, the notification unit is not limited to this, and an audio output unit, such as a speaker that outputs sound, may be employed as the notification unit according to the invention.

In the above-described embodiment, it is employed that there is "an area with a possibility of being an abnormal portion" in an area in a diagnosis image as the given condition according to the invention; however, the condition is not limited to this. It may be employed that an area in a diagnosis image is "an area with a possibility of being an abnormal portion" and there is no "area that is an abnormal portion" in the diagnosis image as the given condition according to the invention.

Modifications 1 to 3 described below may be employed in the above-described embodiment.

### Modification 1

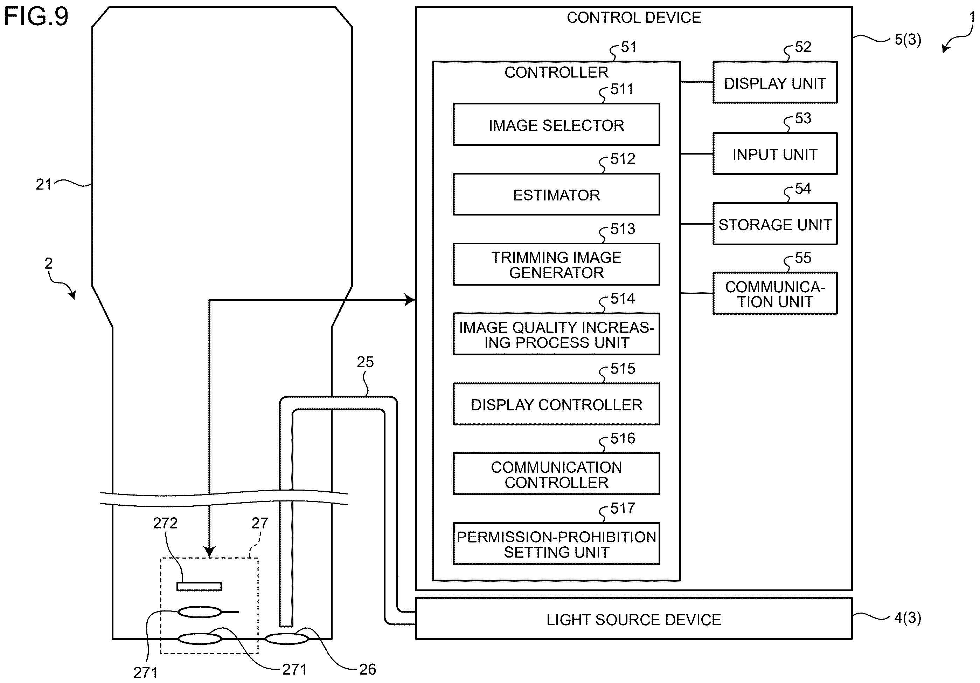

FIG. 9 is a diagram illustrating Modification 1 of the embodiment. Specifically, FIG. 9 corresponds to FIG. 2.

As for the controller 51 according to Modification 1, as illustrated in FIG. 9, a permission-prohibition setting unit 517 is added to the controller 51 described in the above-described embodiment.

According to a user operation of selecting a captured image or a trimming image (or an image-quality-corrected image) on the input unit 53, the image selector 511 according to Modification 1 selects an image that is selected by the user operation as a diagnosis image.

According to a user operation on the input unit 53, the permission-prohibition setting unit 517 sets a state of the image selector 511 at a permitted state or a prohibited state described below.

The permitted state is a state where selecting a diagnosis image by the image selector 511 according to a user operation described above is permitted. In other words, in the permitted state, the image selector 511 selects the image that is selected by the user operation as a diagnosis image.

The prohibited state is a state where selecting a diagnosis image by the image selector 511 according to a user operation described above is prohibited. In other words, in the prohibited state, even when a captured image or a trimming image (or an image-quality-corrected image) is elected by a user operation, the image selector 511 does not select the image that is selected by the user operation as a diagnosis image.

Even in the case where the configuration of Modification 1 is employed, the same effect as that of the above-described embodiment is achieved.

### Modification 2

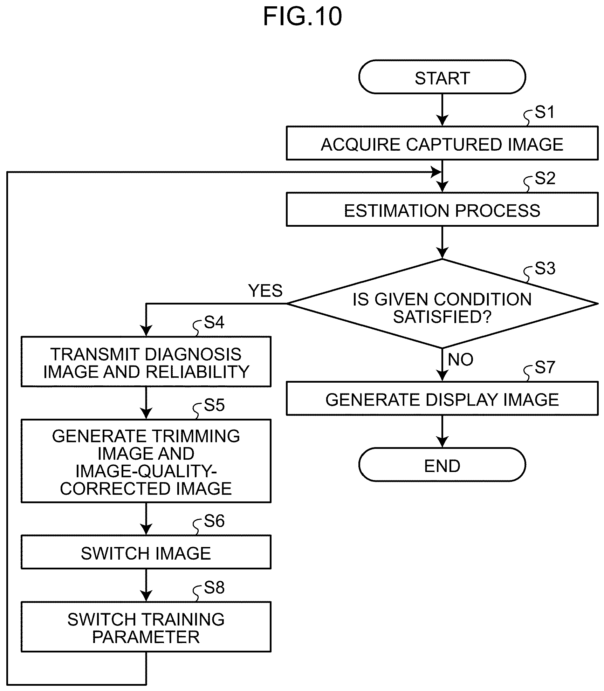

FIG. 10 is a diagram illustrating Modification 2 of the embodiment. Specifically, FIG. 10 is a diagram corresponding to FIG. 4.

In the storage unit 54 according to Modification 2, first to third training parameters described below are stored.

The first training parameter corresponds to the captured image that is acquired at step S1 and is a training parameter of the estimation process trained model that is used to execute the estimation process on the captured image.

The second training parameter corresponds to the trimming image that is generated at step S5 and is a training parameter of the estimation process trained model that is used to execute the estimation process on the trimming image.

The third training parameter corresponds to the image-quality-corrected image that is generated at step S5 and is a training parameter of the estimation process trained model that is used to execute the estimation process on the image-quality-corrected image.

In Modification 2, at step S2, the estimator 512 executes the estimation process on the diagnosis image using the training parameter corresponding to the input diagnosis image among the first to third parameters stored in the storage unit 54. In other words, when the input diagnosis image is the captured image that is acquired at step S1, the estimator 512 sets the first parameter for the training parameter of the estimation process trained model and executes the estimation process on the captured image using. When the diagnosis image is switched to the trimming image or the image-quality-corrected image at step S6, the estimator 512 switches the training parameter from the first training parameter to the second training parameter or the third parameter (step S8). The estimator 512 then sets the second training parameter or the third training parameter for the training parameter of the estimation process trained model and executes the estimation process on the trimming image or the image-quality-corrected image.

According to Modification 2 described above, the following effect is achieved in addition to the same effect as that of the above-described embodiment.

In Modification 2, the estimator 512 executes the estimation process on the diagnosis image using the training parameter corresponding to the input diagnosis image among the first to third training parameters stored in the storage unit 54. Using the training parameter corresponding to the input diagnosis image thus makes it possible to estimate a lesion, or the like, more accurately.

### Modification 3

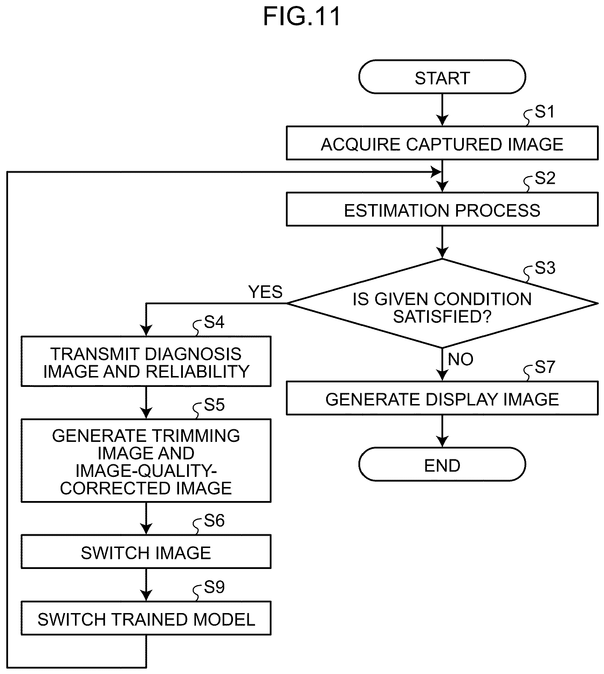

FIG. 11 is a diagram illustrating Modification 3 of the embodiment. Specifically, FIG. 11 is a diagram corresponding to FIG. 4.

First to third trained models described below are stored in the storage unit 54 according to Modification 3.

The first trained model corresponds to the captured image that is acquired at step S1 and is an estimation process trained model that is used to execute the estimation process on the captured image.

The second trained model corresponds to the trimming image that is generated at step S5 and is an estimation process trained model that is used to execute the estimation process on the trimming image.

The third trained model corresponds to the image-quality-corrected image that is generated at step S5 and is an estimation process trained model that is used to execute the estimation process on the image-quality-corrected image.

In Modification 3, at step S2, the estimator 512 executes the estimation process on the diagnosis image using the estimation process trained model corresponding to the input diagnosis image among the first to third trained models stored in the storage unit 54. In other words, in the case where the input diagnosis image is the captured image that is acquired at step S1, the estimator 512 executes the estimation process on the captured image using the first trained model. When the diagnosis image is switched to the trimming image or the image-quality-corrected image at step S6, the estimator 512 switches the estimation process trained model from the first trained model to the second trained model or the third trained model (step S9). The estimator 512 then executes the estimation process on the trimming image or the image-quality-corrected image using the second trained model or the third trained model.

According to Modification 3 described above, the following effect is achieved in addition to the same effect as that of the above-described embodiment.

In Modification 3, the estimator 512 executes the estimation process on the diagnosis image using the estimation process trained model corresponding to the input diagnosis image among the first to third trained models stored in the storage unit 54. Using the estimation process trained model corresponding to the input diagnosis image thus makes it possible to estimate a lesion, or the like, more accurately.

### Reference Signs List

- 1: ENDOSCOPE SYSTEM
- 2: ENDOSCOPE
- 3: PROCESSING DEVICE
- 4: LIGHT SOURCE DEVICE
- 5: CONTROL DEVICE
- 21: INSERTION PORTION
- 22: OPERATION UNIT
- 23: UNIVERSAL CORD
- 24: CONNECTOR
- 25: LIGHT GUIDE
- 26: ILLUMINATION LENS
- 27: IMAGING UNIT
- 51: CONTROLLER
- 52: DISPLAY UNIT
- 53: INPUT UNIT
- 54: STORAGE UNIT
- 55: COMMUNICATION UNIT
- 271: LENS UNIT
- 272: IMAGING DEVICE
- 511: IMAGE SELECTOR
- 512: ESTIMATOR
- 513: TRIMMING IMAGE GENERATOR
- 514: IMAGE QUALITY INCREASING PROCESS UNIT
- 515: DISPLAY CONTROLLER
- 516: COMMUNICATION CONTROLLER
- 517: PERMISSION-PROHIBITION SETTING UNIT
- Ar1, Ar2: AREA
- BD: BED
- F1, F2: DISPLAY IMAGE
- F11: OBSERVATION POSITION IMAGE
- F12, F121: DIAGNOSIS IMAGE
- F122: TRIMMING IMAGE
- F13: IDENTIFICATION INFORMATION
- FT1 TO FT9: THUMBNAIL IMAGE
- OP: OBSERVATION POSITION
- PA: SUBJECT

## Claims

1. An image diagnosis assistance apparatus comprising:
an image selector configured to select, as a diagnosis image, any one of a plurality of images that contain a same subject and on which sets of processing different from one another are executed; and
an estimator configured to, by executing an estimation process on the diagnosis image using a trained model, estimate a diagnostic candidate area serving as a diagnostic candidate in the diagnosis image and output reliability of the diagnostic candidate area,
wherein the image selector is configured to select any one of the plurality of images as the diagnosis image based on the reliability of the diagnostic candidate area.

2. The image diagnosis assistance apparatus according to claim 1, further comprising:
a notification unit configured to make a notification of given information; and
a notification controller configured to cause a notification of the diagnostic candidate area to be made from the notification unit.

3. The image diagnosis assistance apparatus according to claim 1, wherein the plurality of images include at least two of a first captured image obtained by capturing feedback light of light of a first wavelength band from the subject to which the light of the first wavelength band is applied, a trimming image obtained by enlarging a part of the first captured image, an image-quality-corrected image obtained by correcting image quality of the trimming image, and a second captured image obtained by capturing a feedback light of light of a second wavelength band different from the first wavelength band from the subject to which the light of the second wavelength band is applied.

4. The image diagnosis assistance apparatus according to claim 3, wherein the trimming image is an image obtained by enlarging an area containing the diagnostic candidate area in the first captured image.

5. The image diagnosis assistance apparatus according to claim 1, further comprising an operation receiver configured to receive a user operation of selecting any one of the plurality of images,
wherein the image selector is configured to select any one of the plurality of images as the diagnosis image according to the user operation.

6. The image diagnosis assistance apparatus according to claim 5, further comprising a permission-prohibition setting unit configured to set a permitted state where selecting any one of the plurality of images by the image selector according to the user operation is permitted or a prohibited state where the selecting is prohibited.

7. The image diagnosis assistance apparatus according to claim 1, wherein the reliability of the diagnostic candidate area is a value representing accuracy of recognition in the diagnostic candidate area, and
when the reliability of the diagnostic candidate area is under a first threshold and is at or above a second threshold lower than the first threshold, the image selector is configured to switch the diagnosis image that is selected currently to another image of the plurality of images.

8. The image diagnosis assistance apparatus according to claim 1, further comprising
a display unit configured to display a given image; and
a display controller configured to cause the display unit to display the selected diagnosis image.

9. The image diagnosis assistance apparatus according to claim 1, wherein, when the reliability of the diagnostic candidate area satisfies a given condition for a given number of times or for a given time, the image selector is configured to switch the diagnosis image that is selected currently to another image of the plurality of images.

10. The image diagnosis assistance apparatus according to claim 1, further comprising
a communication unit that is connected to an external device such that communication is enabled; and
a communication controller configured to cause the diagnosis image and the reliability of the diagnostic candidate area to be transmitted from the communication unit to the external device.

11. The image diagnosis assistance apparatus according to claim 1, wherein, when the diagnosis image is switched by the image selector, the estimator is configured to switch a training parameter of the trained model that is used for the estimation process to a training parameter corresponding to the switched diagnosis image.

12. The image diagnosis assistance apparatus according to claim 1, wherein, when the diagnosis image is switched by the image selector, the estimator is configured to switch the trained model that is used for the estimation process to a trained model corresponding to the switched diagnosis image.

13. An image diagnosis assistance system comprising:
an imaging device configured to generate a captured image by capturing an image of a subject; and
an image diagnosis assistance apparatus configured to process the captured image,
wherein the image diagnosis assistance apparatus includes:
an image selector configured to select, as a diagnosis image, any one of a plurality of images that contain a same subject and that are obtained by executing sets of processing different from one another are executed on the captured image; and
an estimator configured to, by executing an estimation process on the diagnosis image using a trained model, estimate a diagnostic candidate area serving as a diagnostic candidate in the diagnosis image and output reliability of the diagnostic candidate area, and
the image selector is configured to select any one of the plurality of images as the diagnosis image based on the reliability of the diagnostic candidate area.

14. An image diagnosis assistance method that an image diagnosis assistance apparatus executes, the method comprising:
a step of selecting, as a diagnosis image, any one of a plurality of images that contain a same subject and on which sets of processing different from one another are executed; and
a step of, by executing an estimation process on the diagnosis image using a trained model, estimating a diagnostic candidate area serving as a diagnostic candidate in the diagnosis image and outputting reliability of the diagnostic candidate area,
wherein the step of selecting the diagnosis image includes selecting any one of the plurality of images as the diagnosis image based on the reliability of the diagnostic candidate area.
